# EUROPEAN PATENT SPECIFICATION

(11) **EP 2 572 682 B1**
(45) Date of publication and mention of the grant of the patent: **24.01.2018**
(21) Application number: 12185527.4
(22) Date of filing: 21.09.2012
(51) Int. Cl.: A61F 2/915

(54) **Improved stent geometry**
Verbesserte Stent-Geometrie
Géométrie améliorée d'un stent

(30) Priority: 23.09.2011 US 201113242573
(43) Date of publication of application: 27.03.2013
(73) Proprietor: Cook Medical Technologies LLC, Bloomington, IN 47404 (US)
(72) Inventor: Irwin, Nathaniel A., Co. Limerick (IE); Wooley, Chase B., Floyd Knobs, IN 47119 (US); Boatman, Scott E., Bloomington, IN 47401 (US); Kim, Seoggwan, West Lafayette, IN 47906 (US); Swift, Richard A., South Bend, IN 46635 (US); Tuschka, Jürgen, 92637 Weiden (DE); Kellerer, Daniel, 75179 Pforzheim (DE); Knobloch, Thomas, 76646 Bruchsal (DE)
(74) Representative: Garratt, Peter Douglas

(56) References cited:
- WO-A2-03/030786
- US-A1- 2002 055 770

## Description

### BACKGROUND

The present invention relates generally to medical devices and more particularly to a stent structure.

The use of stents to treat various organs, such as the vascular system, colon, biliary tract, urinary tract, esophagus, trachea and the like, has become common in recent years. Stents are most commonly used to treat blockages, occlusions, narrowing ailments and other similar problems that restrict flow through a passageway. One area where stents are commonly used for treatment involves implanting an endovascular stent into the vascular system in order to improve or maintain blood flow through narrowed arteries. However, stents are also used in other treatments as well, such as the treatment of aneurysms. Stents have been shown to be useful in treating various vessels throughout the vascular system, including both coronary vessels and peripheral vessels (e.g., carotid, brachial, renal, iliac and femoral). In addition, stents have been used in other body vessels as well, such as the digestive tract.

The use of stents in coronary and peripheral vessels has drawn particular attention from the medical community because of the growing number of people each year that suffer from vasculature problems associated with stenosis (i.e., narrowing of a vessel). This has led to an increased demand for medical procedures to treat such problems. The widespread frequency of heart problems and other vasculature problems may be due to a number of societal changes, including the tendency of people to exercise less and the prevalence of unhealthy diets, in conjunction with the fact that people generally have longer life spans now than previous generations. Stents have become a popular alternative for treating vascular stenosis because stenting procedures are considerably less invasive than conventional procedures. For example, stenosis of the coronary arteries was traditionally treated with bypass surgery. In general, bypass surgery involves splitting the chest bone to open the chest cavity and grafting a replacement vessel onto the heart to bypass the blocked, or stenosed, artery. However, coronary bypass surgery is a very invasive procedure that is risky and requires a long recovery time for the patient. Vascular stents are also being more widely used to treat many different peripheral arteries due to the minimally invasive nature of stenting procedures. To address the growing demand for minimally invasive medical procedures for the treatment of coronary arteries, peripheral arteries and other passageway problems, the medical community has begun to turn away from conventional invasive procedures like bypass surgery and increasingly the treatment of choice now involves a variety of stenting procedures.

Many different types of stents and stenting procedures are possible. In general, however, stents are typically designed as tubular support structures that may be inserted percutaneously and transluminally through a body passageway. Traditionally, stents are made from a metal or other synthetic material with a series of radial openings extending through the support structure of the stent to facilitate compression and expansion of the stent. Although stents may be made from many types of materials, including non-metallic materials, common examples of metallic materials that may be used to make stents include stainless steel, nitinol, cobalt-chrome alloys, amorphous metals, tantalum, platinum, gold and titanium. Typically, stents are implanted within a passageway by positioning the stent within the area to be treated and then expanding the stent from a compressed diameter to an expanded diameter. The ability of the stent to expand from a compressed diameter makes it possible to thread the stent to the area to be treated through various narrow body passageways while the stent is in the compressed diameter. Once the stent has been positioned and expanded at the area to be treated, the tubular support structure of the stent contacts and radially supports the inner wall of the passageway. As a result, the implanted stent mechanically prevents the passageway from narrowing and keeps the passageway open to facilitate fluid flow through the passageway.

Stents can generally be characterized as either balloon-expandable or self-expanding. However, stent designs and implantation procedures vary widely. For example, although physicians often prefer particular types of stents for certain types of procedures, the uses for balloon-expandable and self-expanding stents sometimes overlap and procedures related to one type of stent may be adapted to other types of stents.

Balloon-expandable stents are typically used to treat stenosis of the coronary arteries. Usually, balloon-expandable stents are made from ductile materials that plastically deform relatively easily. In the case of stents made from metal, 316L stainless steel that has been annealed is a common choice for this type of stent. One procedure for implanting balloon-expandable stents involves mounting the stent circumferentially on the balloon of a balloon-tipped catheter and threading the catheter through a vessel passageway to the area to be treated. Once the balloon is positioned at the narrowed portion of the vessel to be treated, the balloon is expanded by pumping saline through the catheter to the balloon. As a result, the balloon simultaneously dilates the vessel and radially expands the stent within the dilated portion. The balloon is then deflated and the balloon-tipped catheter is retracted from the passageway. This leaves the expanded stent permanently implanted at the desired location. Ductile metal lends itself to this type of stent since the stent may be compressed by plastic deformation to a small diameter when mounted onto the balloon. When the balloon is later expanded in the vessel, the stent is once again plastically deformed to a larger diameter to provide the desired radial support structure. Traditionally, balloon-expandable stents have been more commonly used in coronary vessels than in peripheral vessels because of the deformable nature of these stents. One reason for this is that peripheral vessels tend to experience frequent traumas from external sources (e.g., impacts to a person's arms, legs, etc.) which are transmitted through the body's tissues to the vessel. In the case of peripheral vessels, there is an increased risk that an external trauma could cause a balloon-expandable stent to once again plastically deform in unexpected ways with potentially severe and/or catastrophic results. In the case of coronary vessels, however, this risk is minimal since coronary vessels rarely experience traumas transmitted from external sources.

Self-expanding stents are increasingly used and accepted by physicians for treating a variety of ailments. Self-expanding stents are usually made of shape memory materials or other elastic materials that act like a spring. Typical metals used in this type of stent include nitinol and 304 stainless steel. A common procedure for implanting a self-expanding stent involves a two-step process. First, the narrowed vessel portion to be treated is dilated with a balloon but without a stent mounted on the balloon. Second, a stent is implanted into the dilated vessel portion. To facilitate stent implantation, the stent is installed on the end of a catheter in a compressed, small diameter state and is usually retained in the small diameter by inserting the stent into a sheath at the end of the catheter. The stent is then guided to the balloon-dilated portion and is released from the catheter by pulling the restraining sheath off the stent. Once released from the restraining sheath, the stent radially springs outward to an expanded diameter until the stent contacts and presses against the vessel wall. Traditionally, self-expanding stents have been more commonly used in peripheral vessels than in coronary vessels due to the shape memory characteristic of the metals that are used in these stents. One advantage of self-expanding stents for peripheral vessels is that traumas from external sources do not permanently deform the stent. Instead, the stent may temporarily deform during an unusually harsh trauma but will spring back to its expanded state once the trauma is relieved. Self-expanding stents, however, are often considered to be less preferred for coronary vessels as compared to balloon-expandable stents. One reason for this is that balloon-expandable stents can be precisely sized to a particular vessel diameter and shape since the ductile metal that is used can be plastically deformed to a desired size and shape. In contrast, self-expanding stents are designed with a particular expansible range. Thus, after being implanted, self-expanding stents continue to exert pressure against the vessel wall.

US 2002/0055770 discloses a stent, comprising: first undulating bands having a first width and second undulating bands having a second width, wherein the first width is greater than the second width.

### SUMMARY

An improved stent structure is described which provides increased radial force. The stent structure has hoop cells and flex cells that alternate along the length of the stent structure. Longitudinal struts connect the hoop cells and flex cells together. The longitudinal struts extend through the hoop cells but do not extend through the flex cells. The flex cells have interconnected flex struts that are wider than interconnected hoop struts in the hoop cells.

The invention may include any of the following aspects in various combinations and may also include any other aspect described below in the written description or in the attached drawings.

A stent, comprising:
an alternating arrangement of hoop cells and flex cells;
the hoop cells comprise a series of first and second longitudinal struts connected at one end to two hoop struts, each of the first and second longitudinal struts extending substantially through an entire length of each of the hoop cells, the first longitudinal struts connected at another end to two flex struts of a proximally adjacent flex cell, and the second longitudinal struts connected at another end to two flex struts of a distally adjacent flex cell;
the flex cells comprise only flex struts interconnected by a series of bends, the first and second longitudinal struts being connected to bends of the series of bends located adjacent the hoop cells through which the first and second longitudinal struts extend, and the first and second longitudinal struts not extending substantially through a length of each of the flex cells;
wherein the hoop struts have a generally uniform first width along an entire length of each of the hoop struts and the flex struts have a generally uniform second width along an entire length of each of the flex struts, the second width being greater than the first width by at least 15% and no more than 25%.

The stent wherein the second width is greater than the first width by at least 18%.

The stent wherein the second width is greater than the first width by about 19%.

The stent wherein the first and second longitudinal struts have a generally uniform third width along an entire length of each of the first and second longitudinal struts, the third width being substantially equal to the first width of the hoop struts.

The stent wherein the second width is about .124 mm and the first width is about .104 mm.

The stent wherein a second overall length of the flex cells is substantially equal to a first overall length of the hoop cells.

The stent wherein the second overall length of the flex cells is about 1.9 mm and the first overall length of the hoop cells is about 1.9 mm.

The stent wherein each of the hoop cells has a number of the hoop struts equal to a number of the flex struts in each of the flex cells.

The stent wherein each of the hoop cells has 18 of the hoop struts and each of the flex cells has 18 of the flex struts.

The stent wherein each of the hoop cells has three first longitudinal struts extending therethrough and three of the second longitudinal struts extending therethrough.

The stent wherein the hoop struts, the first and second longitudinal struts and the flex struts are made from an elastic material, the stent thereby being self-expanding.

The stent wherein the hoop cells and the flex cells are compressible to a compressed diameter such that the stent is deliverable through a 1.333 mm (4 French) delivery sheath and are expandable for use in a body vessel to at least a 5 mm diameter.

The stent wherein the first and second longitudinal struts have a generally uniform third width along an entire length of each of the first and second longitudinal struts, the third width being substantially equal to the first width of the hoop struts, a second overall length of the flex cells is substantially equal to a first overall length of the hoop cells, and each of the hoop cells has a number of the hoop struts equal to a number of the flex struts in each of the flex cells.

The stent wherein each of the hoop cells has three first longitudinal struts extending therethrough and three of the second longitudinal struts extending therethrough, and the second width is greater than the first width by at least 18%.

The stent wherein each of the hoop cells has 18 of the hoop struts and each of the flex cells has 18 of the flex struts, the hoop struts, the first and second longitudinal struts and the flex struts are made from an elastic material, the stent thereby being self-expanding, and the hoop cells and the flex cells are compressible to a compressed diameter such that the stent is deliverable through a 1.333 mm (4 French) delivery sheath and are expandable for use in a body vessel to at least a 5 mm diameter.

The stent wherein the second width is about .124 mm and the first width is about .104 mm, and the second overall length of the flex cells is about 1.9 mm and the first overall length of the hoop cells is about 1.9 mm.

The stent wherein the hoop struts, the first and second longitudinal struts and the flex struts are made from an elastic material, the stent thereby being self-expanding, and the hoop cells and the flex cells are compressible to a compressed diameter such that the stent is deliverable through a 1.333 mm (4 French) delivery sheath and are expandable for use in a body vessel to at least a 5 mm diameter.

The stent wherein each of the hoop cells has 18 of the hoop struts and each of the flex cells has 18 of the flex struts, and each of the hoop cells has three first longitudinal struts extending therethrough and three of the second longitudinal struts extending therethrough.

The stent wherein the second width is greater than the first width by at least 18%.

The stent wherein the second width is greater than the first width by about 19%, the second width is about .124 mm and the first width is about .104 mm, and the second overall length of the flex cells is about 1.9 mm and the first overall length of the hoop cells is about 1.9 mm.

### BRIEF DESCRIPTION OF SEVERAL VIEWS OF THE DRAWINGS

The invention may be more fully understood by reading the following description in conjunction with the drawings, in which:
Figure 1 is a top view of a laid out stent structure;
Figure 2A is a radial compression chart showing test data for stents having flex and hoop struts with equal widths; and
Figure 2B is a radial compression chart showing test data for stents having flex struts that are wider than the hoop struts.

### DETAILED DESCRIPTION

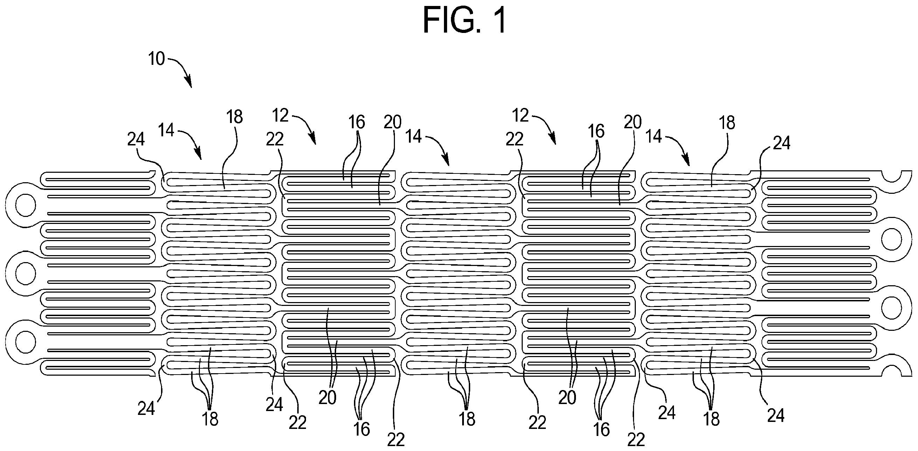

Referring now to the figures, and particularly to Figure 1, a stent structure 10 is shown in a laid out view to show the entire structure 10 of the stent 10. As one of ordinary skill in the art understands, the stent structure 10 shown in Figure 1 may be wrapped into a cylinder to form a cylindrical stent 10, where the top and bottom sides of the stent structure 10 in Figure 1 are preferably integrally connected to each other. Further, one of ordinary skill in the art will recognize that the stent structure 10 shown in Figure 1 is shown in its small diameter, collapsed configuration, which the stent 10 would be compressed to in order to minimally deliver the stent 10 to the desired treatment site. As shown, in this configuration, the struts 16, 18 are compressed so that they are generally parallel to each other and adjacent to the nearest struts 16, 18, 20. However, in the collapsed configuration, there may be a small angular orientation between adjacent struts 16, 18, 20. In the larger diameter, expanded configuration, the hoop struts 16 and the flex struts 18 expand away from each other so that the hoop cells 12 and flex cells 14 form zig-zag rings, with the hoop and flex struts 16, 18 becoming angularly oriented with respect to each other. Thus, whereas adjacent hoop struts 16 and adjacent flex struts 18 are oriented generally parallel to each other in the collapsed configuration, each of the adjacent hoop struts 16 and adjacent flex struts 18 are oriented with acute angles between them in the expanded configuration. However, the longitudinal struts 20 remain generally in the same orientation in both the collapsed and expanded configurations, which as shown in Figure 1 is parallel to the axis of the stent 10. Although the stent structure 10 in Figure 1 may be made from a ductile material so that the stent 10 is balloon-expandable, it may be preferable to make the stent structure 10 from an elastic material so that the stent 10 is self-expanding.

As shown in Figure 1, the stent structure 10 has alternating hoop cells 12 and flex cells 14. The hoop cells 12 have a series of hoop struts 16 connected to each other through a series of hoop bends 22. The hoop cells 12 also have longitudinal struts 20 that extend substantially through the entire length of the hoop cells 12. The longitudinal struts 20 connect the hoop cells 12 and flex cells 14 together so that half of the longitudinal struts 20 connect to the proximally adjacent flex cell 14 and the other half of the longitudinal struts 20 connect to the distally adjacent flex cell 14. Thus, each longitudinal strut 20 is connected at one end to the outside of an adjacent flex cell bend 24, which connects two flex struts 18 together, and is connected at the other end to the inside of a hoop cell bend 22, which connects two hoop struts 16 together. Preferably, each hoop cell 12 has eighteen hoop struts 16 and six longitudinal struts 20. Thus, preferably, there are three hoop struts 16 between adjacent longitudinal struts 20 extending in opposite directions. However, the stent structure 10 may have a different number of longitudinal struts 20 and hoop struts 16. For example, the stent structure 10 may have eight longitudinal struts 20, with four longitudinal struts 20 connected to opposite flex cells 14. The stent structure 10 may also have twenty-four hoop struts 16. Other variations may also be possible.

The flex cells 14 have a series of flex struts 18 connected to each other through a series of bends 24. Because the longitudinal struts 20 do not extend substantially through the length of the flex cells 14, the flex cells 14 only have flex struts 18 within each flex cell 14. Preferably, each flex cell 14 has eighteen flex struts 18. Thus, it is particularly preferable for each of the hoop cells 12 to have the same number of hoop struts 16 as there are flex struts 18 in the flex cells 14. However, it is possible for the flex cells 14 to have a different number of flex struts 18 than the hoop cells 12 have hoop struts 16. For example, the flex cells 14 may have twenty-four flex struts 18 or other variations. Thus, in the preferred embodiment shown in Figure 1, the stent structure 10 has eighteen hoop struts 16 and flex struts per cell 12, 14 and three longitudinal struts 20 per side of each hoop cell 12. Alternatively, the stent structure may have twenty-four hoop struts 16 and flex struts per cell 12, 14 and four longitudinal struts 20 per side of each hoop cell 12. Increases in the number of struts may be useful for larger stents, such as 2 mm (6 French) stents expandable to larger diameters. Other variations are possible as well. It is also preferable for the overall length of the flex cells 14 to be substantially the same as the overall length of the hoop cells 12. For example, overall length of the flex cells 14 and the hoop cells 12 is measured from the outside of opposite bends 22, 24 may be about 1.9 mm. However, it is possible that the overall length of the flex cells 14 may be slightly longer than the overall length of the hoop cells 12, since the extra width of the flex struts 18 may also be applied to the flex strut bends 24. Despite slight differences such as this, the overall lengths of the flex cells 14 and hoop cells 12 are considered to be substantially equal to each other.

In the described stent structure, the width of the flex struts 18 is wider than the width of the hoop struts 16. For example, the width of the flex struts 18 may be about .124 mm, and the width of the hoop struts 16 may be about .104 mm. Thus, the width of the flex struts 18 is greater than the width of the hoop struts 16 by about 19% (i.e., .124 / .104 -1). However, it may also be desirable to have the flex struts 18 be greater than the width of the hoop struts 16 by at least 18% or by at least 15%. Although the flex struts 18 may be as much as 40% wider than the hoop struts 16, it is preferable that the flex struts 18 are no more than 25% wider than the hoop struts 16. The widths of the flex and hoop struts 18, 16 are preferably generally uniform along the length of each strut 18, 16. The flex and hoop struts 18, 16 are also preferably entirely straight from end to end. The width of the longitudinal struts 20 is also preferably generally uniform along the length of each strut 20. The longitudinal struts 20 are also preferably entirely straight from end to end. The width of the longitudinal struts 20 may be substantially equal to the width of the hoop struts 16. The stent structure 10 is preferably cut from a cannula having a uniform thickness with a laser. If desired, the stent structure 10 may be electropolished after being laser cut. Preferably, the final thickness of the struts 16, 18, 20 may be about .14 mm or less, with the hoop struts 16, flex struts 18 and longitudinal struts 20 all having the same thickness of about .14 mm.

One advantage of the stent structure 10 described above is that the average radial force of the stent 10 can be increased compared to a stent where the hoop and flex struts have equal widths. This may be particularly useful for low profile stents that are delivered through particularly small delivery sheaths and yet expand sufficiently to support typically sized body vessels. For example, the described stent structure 10 may be particularly useful for a stent 10 that is deliverable through a 1.333 mm (4 French) delivery sheath and is expandable to at least a 5 mm diameter for use in a body vessel. Low profile stents, such as this example, are typically achieved by making the thickness of the stent structure thinner in order to permit the stent to be compressed down to a particularly small diameter. However, by making the thickness of the struts thinner, the radial force that the stent exerts is reduced when the stent is expanded. This may be detrimental to the performance of the stent when implanted into a body vessel since one purpose of the stent is to exert outward force against the vessel wall to maintain patency. As a result, the described stent structure 10 may be particularly useful for stents 10 with a thickness less than about .15 mm, and more specifically less than about .12 mm.

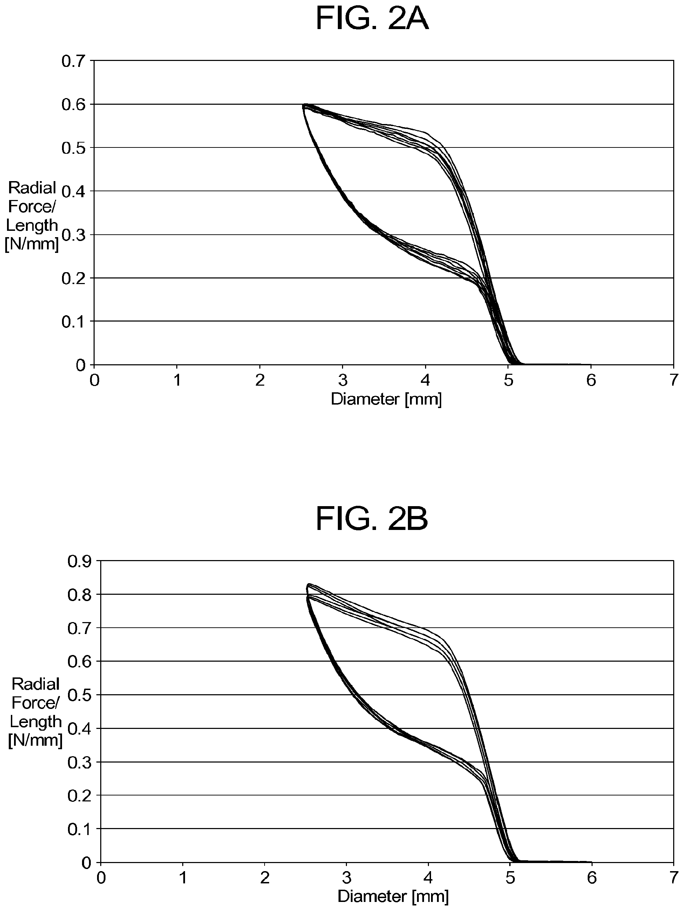

An example of the improved radial force is shown in Figures 2A-2B. In Figure 2A, a radial compression chart is shown with overlapping chart data for 5 mm diameter x 60 mm long stents intended to be delivered through a 4 French sheath. Test data is shown for nitinol stents having a thickness of about .14 mm; hoop, flex and longitudinal struts having equal widths of .104 mm; and different A_{f} temperatures of 18.5°C, 19.2°C and 20.6°C. As shown, the maximum average radial force exerted by the stents is about 0.6 N/mm. In Figure 2B, a radial compression chart is shown with overlapping chart data for 5 mm x 60 mm long stents with the stent structure 10 described above and intended to be delivered through a 4 French sheath. Test data is shown for two nitinol stents having a thickness of about .14 mm; hoop and longitudinal struts 16, 20 having equal widths of .104 mm; flex struts 18 having a width of .124 mm; and an A_{f} temperature of 18.5°C. As shown, the maximum average radial force exerted by the stents is about 0.8 N/mm. Thus, the improved stent structure 10 provides a significant improvement in radial force compared to a stent structure with hoop and flex struts of equal width.

However, increasing the width of the flex struts 18 reduces the flexibility of the stent 10. As a result, one of ordinary skill in the art may not immediately consider the improved stent 10 to be an optimal design. In fact, one of ordinary skill in the art may find it to be counterintuitive to increase the width of the flex struts 18 because the flex cells 14 are specifically designed in this particular stent arrangement to provide increased flexibility to the stent 10. By contrast, the hoop cells 12 are specifically designed to provide increased radial force but are less flexible than the flex cells 14. Thus, it would typically be undesirable to increase the width of the flex struts 18 because this would change the intended purpose of the flex struts 18 from providing increased flexibility and less radial force to providing more radial force but less flexibility.

For example, increasing the width of the flex struts 18 in a 5 mm stent that is intended to be delivered through a 1.333 mm (4 French) sheath results in a stiffness ratio of 1.28 for axial compression, 1.22 for bending, and 1.23 for torsion, when compared with the same stent structure but without the wider flex struts 18. Thus, the improved stent 10 will be significantly stiffer than a stent without wider flex struts 18. This may make the stent less trackable during delivery through tortuous arteries; less conformable to the vessel wall during or after implantation; and may change the fatigue life of the stent 10.

While the described wider flex struts 18 are most preferred in a low profile stent like the 1.333 mm (4 French) stent described, it may be possible to increase the width of the flex struts in other stents as well. For example, in a 1.667 mm (5 French) stent with the same structural arrangement shown in Figure 1 that is expandable to 8 mm, the width of the flex struts may be about .132 mm, and the width of the hoop struts may be about .125 mm. Thus, the width of the flex struts may be about 5.6% (i.e., .132 / .125 - 1) greater than the width of the hoop struts. The width of the flex struts in a 1.667 mm (5 French) stent may also be greater than the width of the hoop struts by as little as 1% and as much as 10% if desired. In a 1.667 mm (5 French) stent as described, it is also preferable for the final thickness of the struts to be about .16 mm to about .2 mm, or more preferably about .18 mm to about .19 mm.

While preferred embodiments of the invention have been described, it should be understood that the invention is not so limited, and modifications may be made without departing from the invention. The scope of the invention is defined by the appended claims, and all devices that come within the meaning of the claims, are intended to be embraced therein. Furthermore, the advantages described above are not necessarily the only advantages of the invention, and it is not necessarily expected that all of the described advantages will be achieved with every embodiment of the invention.

## Claims

1. A stent, comprising:
an alternating arrangement of hoop cells and flex cells;
said hoop cells comprise a series of first and second longitudinal struts connected at one end to two hoop struts, each of said first and second longitudinal struts extending substantially through an entire length of each of said hoop cells, said first longitudinal struts connected at another end to two flex struts of a proximally adjacent flex cell, and said second longitudinal struts connected at another end to two flex struts of a distally adjacent flex cell;
said flex cells comprise only flex struts interconnected by a series of bends, said first and second longitudinal struts being connected to bends of said series of bends located adjacent said hoop cells through which said first and second longitudinal struts extend, and said first and second longitudinal struts not extending substantially through a length of each of said flex cells;
wherein each of said hoop cells has a number of said hoop struts equal to a number of said flex struts in each of said flex cells;
wherein a second overall length of said flex cells is substantially equal to a first overall length of said hoop cells;
wherein said hoop cells and said flex cells are adapted to be compressible to a compressed diameter such that said stent is deliverable through a 1.333 mm (4 French) delivery sheath and are expandable for use in a body vessel to at least a 5 mm diameter
wherein the thickness of the stent is less than about. 12 mm; wherein said hoop struts have a generally uniform first width along an entire ength of each of said hoop struts and said flex struts have a generally uniform second width along an entire length of each of said flex struts, said second width being greater than said first width by at least 15% and no more than 25%.

2. The stent according to claim 1, wherein said second width is greater than said first width by at least 18%.

3. The stent according to claim 1, wherein said second width is greater than said first width by about 19%.

4. The stent according to any one of the preceding claims, wherein said first and second longitudinal struts have a generally uniform third width along an entire length of each of said first and second longitudinal struts, said third width being substantially equal to said first width of said hoop struts.

5. The stent according to any one of the preceding claims, wherein said second width is about .124 mm and said first width is about .104 mm.

6. The stent according to any one of the preceding claims, wherein said second overall length of said flex cells is about 1.9 mm and said first overall length of said hoop cells is about 1.9 mm.

7. The stent according to any one of the preceding claims, wherein each of said hoop cells has 18 of said hoop struts and each of said flex cells has 18 of said flex struts.

8. The stent according to any one of the preceding claims, wherein each of said hoop cells has three first longitudinal struts extending therethrough and three of said second longitudinal struts extending therethrough.

9. The stent according to any one of the preceding claims, wherein said hoop struts, said first and second longitudinal struts and said flex struts are made from an elastic material, said stent thereby being self-expanding.

## Patentansprüche

1. Stent, umfassend:
eine alternierende Anordnung von Reifenzellen und Biegezellen;
wobei die Reifenzellen eine Reihe von ersten und zweiten Längsstreben umfassen, die an einem Ende mit zwei Reifenstreben verbunden sind, wobei sich jede der ersten und zweiten Längsstreben im Wesentlichen durch eine gesamte Länge jeder der Reifenzellen erstreckt, wobei die ersten Längsstreben an einem anderen Ende mit zwei Biegestreben einer proximal benachbarten Biegezelle verbunden sind und die zweiten Längsstreben an einem anderen Ende mit zwei Biegestreben einer distal benachbarten Biegezelle verbunden sind;
wobei die Biegezellen nur Biegestreben umfassen, die durch eine Reihe von Biegungen miteinander verbunden sind, wobei die ersten und zweiten Längsstreben mit Biegungen der Reihe von Biegungen verbunden sind, die neben den Reifenzellen angeordnet sind, durch die sich die ersten und zweiten Längsstreben erstrecken, und wobei sich die ersten und zweiten Längsstreben nicht im Wesentlichen durch eine Länge jeder der Biegezellen erstrecken;
wobei jede der Reifenzellen eine Anzahl der Reifenstreben aufweist, die einer Anzahl der Biegestreben in jeder der Biegezellen gleicht;
wobei eine zweite Gesamtlänge der Biegezellen im Wesentlichen einer ersten Gesamtlänge der Reifenzellen gleicht;
wobei die Reifenzellen und die Biegezellen ausgelegt sind, zu einem komprimierten Durchmesser komprimierbar zu sein, derart, dass der Stent durch eine Einführungshülle von 1,333 mm (4 French) abgegeben werden kann und zur Verwendung in einem Körpergefäß auf einen Durchmesser von mindestens 5 mm expandierbar ist,
wobei die Dicke des Stents weniger als ungefähr 0,12 mm beträgt,
wobei die Reifenstreben eine allgemein gleichmäßige erste Breite entlang einer gesamten Länge jeder der Reifenstreben aufweisen und die Biegestreben eine allgemein gleichmäßige zweite Breite entlang einer gesamten Länge jeder der Biegestreben aufweisen, wobei die zweite Breite mindestens 15% und höchstens 25% größer als die erste Breite ist.

2. Stent nach Anspruch 1, wobei die zweite Breite mindestens 18% größer als die erste Breite ist.

3. Stent nach Anspruch 1, wobei die zweite Breite ungefähr 19% größer als die erste Breite ist.

4. Stent nach einem der vorhergehenden Ansprüche, wobei die ersten und zweiten Längsstreben eine allgemein gleichmäßige dritte Breite entlang einer gesamten Länge jeder der ersten und zweiten Längsstreben aufweisen, wobei die dritte Breite im Wesentlichen der ersten Breite der Reifenstreben gleicht.

5. Stent nach einem der vorhergehenden Ansprüche, wobei die zweite Breite ungefähr 0,124 mm beträgt und die erste Breite ungefähr 0,104 mm beträgt.

6. Stent nach einem der vorhergehenden Ansprüche, wobei die zweite Gesamtlänge der Biegezellen ungefähr 1,9 mm beträgt und die erste Gesamtlänge der Reifenzellen ungefähr 1,9 mm beträgt.

7. Stent nach einem der vorhergehenden Ansprüche, wobei jede der Reifenzellen 18 der Reifenstreben aufweist und jede der Biegezellen 18 der Biegestreben aufweist.

8. Stent nach einem der vorhergehenden Ansprüche, wobei jede der Reifenzellen drei erste Längsstreben, die sich durch sie hindurch erstrecken, und drei der zweiten Längsstreben, die sich durch sie hindurch erstrecken, aufweist.

9. Stent nach einem der vorhergehenden Ansprüche, wobei die Reifenstreben, die ersten und zweiten Längsstreben und die Biegestreben aus einem elastischen Material gefertigt sind, wobei der Stent dadurch selbst-expandierend ist.

## Revendications

1. Endoprothèse vasculaire comprenant :
un agencement en alternance de cellules en arceau et de cellules flexibles ;
lesdites cellules en arceau comprennent une série de premières et de secondes traverses longitudinales raccordées à une extrémité aux deux traverses en arceau, chacune desdites premières et secondes traverses longitudinales s'étendant sensiblement sur toute la longueur de chacune desdites cellules en arceau, lesdites premières traverses longitudinales étant raccordées à une autre extrémité aux deux traverses flexibles d'une cellule flexible adjacente proximalement, et lesdites secondes traverses longitudinales étant raccordées à une autre extrémité aux deux traverses flexibles d'une cellule flexible adjacente distalement ;
lesdites cellules flexibles ne comprennent que des traverses flexibles raccordées entre elles par une série de coudes, lesdites premières et secondes traverses longitudinales étant raccordées aux coudes de ladite série de coudes situés tout à côté desdites cellules en arceau à travers lesquelles s'étendent lesdites premières et secondes traverses longitudinales, et lesdites premières et secondes traverses longitudinales ne s'étendant pas sensiblement à travers la longueur de chacune desdites cellules flexibles ;
dans laquelle chacune desdites cellules en arceau comporte un nombre desdites traverses en arceau égal au nombre desdites traverses flexibles dans chacune desdites cellules flexibles ;
dans laquelle la seconde longueur totale desdites cellules flexibles est sensiblement égale à la première longueur totale desdites cellules en arceau ;
dans laquelle lesdites cellules en arceau et lesdites cellules flexibles sont aptes à pouvoir être comprimées à un diamètre comprimé tel que ladite endoprothèse peut être mise en place par une gaine de mise en place de 1,333 mm (calibre 4F) et peuvent se dilater pour être utilisés dans un vaisseau corporel jusqu'à au moins 5 mm de diamètre ;
dans laquelle l'épaisseur de l'endoprothèse est inférieure à environ 0,12 mm ;
dans laquelle lesdites traverses en arceau ont une première largeur dans l'ensemble uniforme le long de toute la longueur de chacune desdites traverses en arceau et dans laquelle lesdites traverses flexibles ont une deuxième largeur dans l'ensemble uniforme le long de toute la longueur de chacune desdites traverses flexibles, ladite deuxième largeur étant supérieure à ladite première largeur d'au moins 15 % et de pas plus de 25 %.

2. Endoprothèse selon la revendication 1, dans laquelle ladite deuxième largeur est supérieure à ladite première largeur d'au moins 18 %.

3. Endoprothèse selon la revendication 1, dans laquelle ladite deuxième largeur est supérieure à ladite première largeur d'environ 19 %.

4. Endoprothèse selon l'une quelconque des revendications précédentes, dans laquelle lesdites premières et secondes traverses longitudinales ont une troisième largeur dans l'ensemble uniforme le long de toute la longueur de chacune desdites premières et secondes traverses longitudinales, ladite troisième largeur étant sensiblement égale à ladite première largeur desdites traverses en arceau.

5. Endoprothèse selon l'une quelconque des revendications précédentes, dans laquelle ladite deuxième largeur est d'environ 0,124 mm et ladite première largeur est d'environ 0,104 mm.

6. Endoprothèse selon l'une quelconque des revendications précédentes, dans laquelle ladite seconde longueur totale desdites cellules flexibles est d'environ 1,9 mm et ladite première longueur totale desdites cellules en arceau est d'environ 1,9 mm.

7. Endoprothèse selon l'une quelconque des revendications précédentes, dans laquelle chacune desdites cellules en arceau comporte 18 desdites traverses en arceau et chacune desdites cellules flexibles comporte 18 desdites traverses flexibles.

8. Endoprothèse selon l'une quelconque des revendications précédentes, dans laquelle chacune desdites cellules en arceau comporte trois premières traverses longitudinales qui la traversent et trois desdites secondes traverses longitudinales qui la traversent.

9. Endoprothèse selon l'une quelconque des revendications précédentes, dans laquelle lesdites traverses en arceau, lesdites premières et secondes traverses longitudinales et lesdites traverses flexibles sont faites d'une matière élastique, ladite endoprothèse étant de ce fait auto-dilatable.
